# EUROPÄISCHE PATENTSCHRIFT

(11) **EP 1 315 520 B1**
(45) Veröffentlichungstag und Bekanntmachung des Hinweises auf die Patenterteilung: **04.05.2005**
(21) Anmeldenummer: 01982241.0
(22) Anmeldetag: 04.09.2001
(51) Int. Cl.: A61K 39/395, A61K 39/00, A61P 35/00

(54) **VERWENDUNG VON TRIFUNKTIONELLEN BISPEZIFISCHEN UND TRISPEZIFISCHEN ANTIKÖRPERN ZUR BEHANDLUNG VON MALIGNEM ASZITES**
USE OF TRIFUNCTIONAL BISPECIFIC AND TRISPECIFIC ANTIBODIES FOR THE TREATMENT OF MALIGNANT ASCITES
UTILISATION D'ANTICORPS TRIFONCTIONNELS BISPECIFIQUES ET TRISPECIFIQUES DANS LE TRAITEMENT D'ASCITES MALIGNES

(30) Priorität: 04.09.2000 DE 10043437
(43) Veröffentlichungstag der Anmeldung: 04.06.2003
(73) Patentinhaber: Lindhofer, Horst, Dr., 82194 Gröbenzell (DE)
(72) Erfinder: Lindhofer, Horst, Dr., 82194 Gröbenzell (DE)
(74) Vertreter: Reinhard - Skuhra - Weise & Partner GbR
(86) Internationale Anmeldenummer: PCT/EP2001/010184
(87) Internationale Veröffentlichungsnummer: WO 2002/020039

(56) Entgegenhaltungen:
- DE-A- 19 859 110
- DE-A- 19 859 115
- H. LINDHOFER: "Neuartige trifunktionelle Antikörper befreien Stammzelltransplantate von residualen Tumorzellen." BIOSPEKTRUM, Bd. 6, Nr. 6, 2000, Seiten 500-501, XP001053756 Deutschland
- R. ZEIDLER ET AL.: "The Fc-region of a new class of intact bispecific antibody mediates activation of accessory cells and NK cells and induces direct phagocytosis of tumour cells." BRITISH JOURNAL OF CANCER, Bd. 83, Nr. 2, Juli 2000 (2000-07), Seiten 261-266, XP001053758 Basingstoke, Grossbritannien
- H. LINDHOFER ET AL.: "Preferential species-restricted heavy/light chain pairing in rat/mouse quadromas. Implications for a single-step purification of bispecific antibodies." THE JOURNAL OF IMMUNOLOGY, Bd. 155, Nr. 1, 1. Juli 1995 (1995-07-01), Seiten 219-225, XP002190775 Baltimore, MD, USA
- G. STRAUSS ET AL.: "Without prior stimulation, tumor-associated lymphocytes from malignant effusions lyse autologous tumor cells in the presence of bispecific antibody HEA125xOKT3." CLINICAL CANCER RESEARCH, Bd. 5, Nr. 1, Januar 1999 (1999-01), Seiten 171-180, XP002190776 Philadelphia, PA, USA

## Beschreibung

Die Erfindung betrifft die Verwendung trifunktioneller bispezifische und/oder trispezifische Antikörper zur Herstellung einer pharmazeutischen Zubereitung zur Zerstörung der Tumorzellen, in der Aszites flüssigkeit und/oder in einem Pleuraerguß, um malignen Aszites und einen Pleuraerguß zu behandeln.

Maligner Aszites kann von einer Reihe von Primärtumoren wie z.B. Brustkrebs, Ovariatkarzinom oder den gastrointestinalen Karzinomen hervorgerufen werden. Obwohl Aszites bei einem hohen Prozentsatz der Patienten bereits bei der Erstmanifestation einer Tumorerkrankung nachgewiesen wird, ist er ein Anzeichen für eine fortgeschrittene Erkrankung.

Die gegenwärtigen Therapiemöglichkeiten für Aszites sind u. A. Punktion, örtliche Chemotherapie oder diuretische Behandlung. Alle diese Optionen haben gravierende Nachteile; so führt die Punktion nur zu einer kurzfristigen Erleichterung und es muß im Durchschnitt nach 9,5 Tagen erneut punktiert werden (Mackey et al., J. Pain Symptom Manage., 19:193, 2000). Die Chemotherapie dagegen kann nur bei den Patienten ansprechen, die nicht bereits Chemotherapie-resistente Tumorzellen entwickelt haben, was leider oft der Fall ist. Insofern besteht für Aszites bei Tumorerkrankungen ein enormer Bedarf für die Verbesserung der klinischen Behandlungsmöglichkeiten.

Ähnlich ist die Situation beim durch Tumorzellen induzierten Pleuraerguß. Der Pleuraerguß ist eine Flüssigkeitsansammlung im Brustfellraum und kann sich nach Punktion erneut bilden. Da eine der Ursachen für den Pleuraerguß das Vorhandensein von Tumorzellen in diesem Kompartiment ist, ist die Zerstörung der Tumorzellen eine wesentliche Voraussetzung für die Unterbindung der Neubildung des Pleuraergusses. D.h. die Problematik und die damit verbundenen Lösungsansätze sind beim malignen Aszites und dem auf Tumorzellen basierendem Pleuraerguß sehr ähnlich.

Es ist eine Aufgabe der Erfindung, ein neues Mittel zur Behandlung von malignem Aszites und zur Behandlung eines Pleuraergusses bereitzustellen, welches die Nachteile des Standes der Technik überwindet, insbesondere eine spürbare Erleichterung des Patienten bietet.

Diese Aufgabe wird erfindungsgemäß durch die Verwendung eines trifunktionellen bispezifischen und/oder trispezifischen Antikörpers mit den nachfolgenden Eigenschaften gelöst:
a) bindet an eine T-Zelle;
b) bindet an zumindest ein Antigen auf einer Tumorzelle, die mit malignem Aszites und/oder einem Pleuraerguß in Zusammenhang steht;
c) bindet durch seinen Fc-Teil (bei bispezifischen Antikörpern) oder durch eine dritte Spezifität (bei trispezifischen Antikörpern) an Fc-Rezeptor positive Zellen zur Herstellung einer pharmazeutischen Zubereitung zur Behandlung von malignem Aszites und/oder in der Aszitesflüssigkeit und/oder im Pleuraerguß.

Weitere Ausgestaltungen der Erfindung sind den beiliegenden Patentansprüchen sowie der nachfolgenden Beschreibung zu entnehmen. Es sei darauf hingewiesen, das die Erfindung nicht auf die nachfolgend genannten bevorzugten Ausgestaltungen und die Beispiele beschränkt ist. Vielmehr kann der Fachmann im Rahmen seines Fachwissens die Erfindung im Umfang der beiliegenden Ansprüche in Verbindung mit der Beschreibung abwandeln, ohne von der Erfindung abzuweichen.

Die beiliegenden Abbildungen dienen zur weiteren Veranschaulichung der Erfindung. Die Abbildungen zeigen:
- Abbildung 1:: Elimination von EpCAM+ Tumorzellen und Proliferation von Immunzellen im Aszites unter bsAk-Therapie;
- Abbildung 2:: FACS-Analyse von Tumorzellen auf Oberflächenantigene;
- Abbildung 3:: Zerstörung von residuellen Tumorzellen nach CD3 x EpCAM-Behandlung mittels RT-PCR;
- Abbildung 4:: Nachweis von EpCAM positiven Tumorzellen aus der Aszitesflüssigkeit in der Durchflußzytometrie im Verlauf der Antikörpertherapie;
- Abbildung 5:: Abnahme der Aszites-Flüssigkeitsbildung unter der Therapie mit den Antikörpern REMOVAB (anti EpCAM x anti CD3) und REXOMAB (anti Her2/neu x anti CD3).

Trifunktionelle bispezifische und trispezifische Antikörper und verschiedene Einsatzbereiche dieser Antikörper gehören zum Stand der Technik. Beispielsweise sei hier hingewiesen auf die DE 198 44 157.6 sowie die DE 199 25 586. Dieser Stand der Technik beschreibt zwar die Verwendung bispezifischer und trispezifischer Antikörper mit den drei Merkmalen a, b und c zur Immunisierung gegen Tumoren; eine direkte Behandlungsmöglichkeit der Tumoren durch Zerstörung der Tumorzellen, die über eine Induktion einer Immunität hinausreicht, wird jedoch in diesen Dokumenten nicht beschrieben. Insbesondere wird durch diese Druckschriften nicht ausgeführt, das ein höchst spezieller Teilbereich der ungeheuer breiten Palette an Tumorerkrankungen, nämlich der malignen Aszites, aber auch ein Pleuraerguß, durch trifunktionelle bispezifische und trispezifische Antikörper mit den vorstehend aufgeführten Merkmalen in höchst effizienter und nicht vorhersehbarer Weise behandelbar ist. Ein Behandlungserfolg konnte bereits bei zwei Patienten in der Klinik, die an einer finalen Tumorerkrankung litten, erreicht werden (vgl. Beispiel 1 und 2).

Die pharmazeutische Zubereitung ist so ausgelegt, daß sie zumindest eine Primärimmunisierung durch Zerstörung der Tumorzellen erreicht. Hierzu wird die pharmazeutische Zubereitung, welche die nachfolgend näher beschriebenen trifunktionellen Antikörper enthält, in mehreren Dosen verabreicht. Die erste Dosis wird so gewählt, daß eine Fremdproteinreaktion des Patienten gegen den verabreichten Antikörper getestet wird. Die zweite Dosis wird bevorzugt so gewählt, daß die Immunzellen des Patienten aktiviert werden und proliferieren. Eine dritte Dosis und weitere Dosen werden so gewählt, daß sie insbesondere zur Zerstörung der Tumorzellen führen.

Die Verabreichung der ersten Dosis erfolgt in einer Menge von 1 bis 20 µg, bevorzugt 5 bis 10 µg des Antikörpers, um allergische Reaktionen zu testen. Die Verabreichung der zweiten Dosis erfolgt in einer Menge von 20 bis 80 µg, bevorzugt bis 100 µg, weiterhin bevorzugt 30 bis 60 µg an Antikörper. Die Verabreichung der dritten Dosis und jeder weiteren Dosis erfolgt in einer Menge von über 80 µg, bevorzugt 100 bis 500 µg, weiterhin bevorzugt 100 bis 300 µg des Antikörpers. Es ist für einen Fachmann selbstverständlich, daß diese Mengenangaben Richtwerte darstellen, die von Patient zu Patient und von Antikörper zu Antikörper durchaus unterschiedlich sein können. Es gehört jedoch zum Fachwissen eines Mediziners, die individuell einzusetzende Antikörpermenge entsprechend auszuwählen.

Die Zahl der Applikationen liegt bevorzugt bei einer 3 bis 8, bevorzugt 3 bis 6-maligen Verabreichung einer Dosis. Die Verabreichung erfolgt in gewissen zeitlichen Abständen, wobei insbesondere 48 bis 72 Stunden gewählt werden. Bevorzugt wird zwischen den einzelnen Applikationen ein Zeitraum von 2 - 3 Tagen plus minus mehrere Stunden gewählt. Die Applikationen werden bevorzugt über einen Zeitraum von 12 - 14 Tagen fortgesetzt. Der Zeitraum wird so gewählt, daß bei Auftreten erster Immunreaktionen gegen das Antikörperprotein weitere Antikörperdosen nicht mehr verabreicht werden. Bei Verwendung eines Mausantikörpers wird beispielsweise die HAMA (human anti mouse antibody)-Reaktion bestimmt, die im Allgemeinen nach 16 bis 21 Tagen einsetzt, bei einigen Patienten früher, bei anderen später oder überhaupt nicht.

Maligner Aszites ist, wie oben ausgeführt, besonders häufig bei einer Reihe von Primärtumoren, wie zum Beispiel Brustkrebs, Ovarialkarzinomen und gastrointestinalen Karzinomen. Derartige Tumorerkrankungen werden zunächst durch chirurgische Entfernung des Tumorgewebes und anschließende Chemotherapie behandelt. In einer bevorzugten Ausführungsform der Erfindung werden Immunzellen des Patienten durch Apherese gewonnen. Ein derartiges Aphereseprodukt ist eine Sammlung von Blutzellen, die insbesondere keine Erythrozyten enthält. Enthalten sind insbesondere T-Zellen, Makrophagen, Monozyten, NK-Zellen, dendritische Zellen, Granulozyten und B-Zellen. Die Gewinnung des Apherese-Produkts muß insbesondere vor der ersten Chemotherapie erfolgen, um eine Unversehrtheit der Immunzellen zu gewährleisten, die praktisch immer durch die Chemotherapeutika in Mitleidenschaft gezogen werden.

In einer Ausgestaltung der Erfindung wird der Patient nicht nur mit den trifunktionellen bispezifischen und/oder trispezifischen Antikörpern behandelt, sondern er erhält im Rahmen der Sekundärimmunisierung zusätzlich inaktivierte autologe und/oder allogene Tumorzellen und autologe Immunzellen. Bei den autologen Immunzellen handelt es sich bevorzugt um T-Lymphozyten und akzessorische Zellen, beispielsweise Monozyten, Makrophagen und dendritische Zellen. Diese sind beispielsweise in PBMC (peripheral blood mononuclear cells) enthalten, die beispielsweise aus heparinisientem Blut über Ficoll-Dichtegradientzentrifugation gewonnen werden. Es ist aber auch möglich, Subfraktionen einzusetzen, beispielsweise lediglich die T-Lymphozyten und dendritische Zellen bzw. Makrophagen usw. In einer bevorzugten Ausführungsform handelt es sich bei den autologen Immunzellen um die Apheresezellen, die zusammen mit dem Antikörper und den inaktivierten autologen oder allogenen Tumorzellen vermischt eingesetzt werden.

Zur erfolgreichen Immunisierung ist es weiterhin vorteilhaft, daß die Menge der verabreichten Tumorzellen in geeigneter Weise ausgewählt wird. So konnte in den Versuchen im murinen Tumormodell gezeigt werden, daß eine zu geringe Anzahl an Tumorzellen nicht den gewünschten Immunisierungserfolg bringt. Eine zu große Menge an Tumormaterial wiederum kann sich nachteilig auswirken, da beispielsweise Toleranzphänomene auftreten können. Überträgt man diese Resultate auf die Situation im Patienten, heißt dies, daß es für den Immunisierungserfolg vorteilhaft ist, eine definierte Menge an Tumormaterial im korrekten räumlichen Kontext mit einer ebenfalls definierten Menge an Antikörpern zu verabreichen. Ein Immunisierungserfolg stellt sich zwar auch dann ein, wenn einer dieser Parameter nicht optimiert wurde; besonders gute Ergebnisse werden jedoch erzielt, wenn sowohl die Mengen des Antikörpers als auch die Menge an Tumormaterial als auch der räumliche Kontext aufeinander abgestimmt und optimiert wurden.

Unter Berücksichtigung der obigen Ausführungen kann bei einer zu geringen Tumorzellzahl beispielsweise nur ein unzureichender Immunschutz etabliert werden. Daher ist es für einen vollständigen Immunisierungserfolg notwendig, mit einer definierten Anzahl an inaktivierten Tumorzellen und einer definierten Menge an bispezifischen und/oder trispezifischen Antikörpern zu immunisieren. Die jeweiligen Zahlen können vom Fachmann anhand von Versuchen etabliert werden.

Die Tumorzellen werden bevorzugt in einer Menge von 10⁵ - 10⁸ Zellen pro Applikation verabreicht, wobei sich eine Zellzahl von ca. 10⁷ als bevorzugt herausgestellt hat. Die Tumorzellen wurden, wie oben ausgeführt, vor der Reapplikation so behandelt, daß ihr Überleben nach Reapplikation verhindert wird, wobei sie wahlweise zusätzlich einer Hitzevorbehandlung unterzogen werden können.

Die Tumorzellen werden aus Tumormaterial des zu behandelnden Patienten gewonnen. Das Tumormaterial wird beispielsweise durch enzymatische Behandlung, bevorzugt durch Kollagenase-Behandlung, zu einer Einzelzellsuspension aufbereitet. Wichtig ist, daß die Tumorzellen möglichst unbeschädigt vorliegen. Lysate der Tumorzellen haben sich als ungeeignet herausgestellt. Die Tumorzellen werden vor ihrer Applikation derart behandelt, daß ein Überleben der Tumorzellen im Patienten ausgeschlossen wird. Die Tumorzellen werden deshalb in an sich bekannter Weise behandelt, beispielsweise durch Bestrahlung oder durch Behandlung mit chemischen Agenzien. Durch die Behandlung soll insbesondere auch die äußere Struktur der Tumorzelle unverändert bleiben, so daß das Erkennungsmuster für die Antikörper erhalten bleibt.

Zur Bestrahlung wird bevorzugt eine γ-Bestrahlung eingesetzt, die vorzugsweise mit einer Dosis von 20 bis 200 Gy erfolgt. Bei einer chemischen Behandlung hat sich Mitomycin C besonders bewährt.

Eine weitere Verbesserung der Immunogenität der Tumorzellen kann dadurch erreicht werden, daß diese vor Infusion einer Hitzevorbehandlung unterzogen werden. Die Temperatur liegt in einem Bereich von ca. 40 bis 45°C, wobei ein Bereich von 41 bis 42°C bevorzugt wird. Das Optimum kann durch Versuche ermittelt werden. Bevorzugte Ergebnisse werden insbesondere bei einer Temperatur von ca. 41,8°C erreicht. Der Zeitraum für die Hitzevorbehandlung beträgt im allgemeinen 1 bis 6 Stunden, bevorzugt ca. 3 Stunden. Sowohl der Zeitraum als auch die Temperatur, die optimalerweise eingesetzt werden, hängen auch von der Art des zu behandelnden Tumors ab. Die jeweiligen Optima können vom Fachmann anhand von Versuchen ermittelt werden.

Die Verabreichung der einzelnen Dosen erfolgt im Allgemeinen so, daß zur Erzielung einer Primärimmunisierung, d. h. zur Zerstörung der Tumorzellen, eine intraperitoneale Applikationsform gewählt wird, bei der die Antikörper in den Patienten infundiert werden. Die Verabreichung der Dosen für eine Sekundärimmunisierung erfolgt im Allgemeinen durch eine örtliche Applikation, beispielsweise intradermal, subkutan oder intramuskulär. Sie kann aber auch intraperitoneal oder intravenös erfolgen.

Wenn zusammen mit den trifunktionellen Antikörper auch die inaktivierten autologen und/oder allogenen Tumorzellen und die autologen Immunzellen zur Erzielung einer Sekundärimmunität verabreicht werden, werden bevorzugt die nachfolgenden Mengen eingesetzt:
a) 1-200 x 10⁶ inaktivierte Tumorzellen, bevorzugt 10-100 x 10⁶ inaktivierte Tumorzellen
b) 10-500 x 10⁶ Immunzellen, bevorzugt 50-200 x 10⁶ Immunzellen
c) 1-10 µg trifunktionelle bispezifische Antikörper oder trispezifische Antikörper, bevorzugt 2-5 µg Antikörper.

Wie bereits oben ausgeführt wird jedoch der Fachmann die jeweilige Menge und den Applikationsort so wählen, daß ein optimaler therapeutischer Erfolg gewährleistet ist.

Die erfindungsgemäß bereitgestellte pharmazeutische Zubereitung liegt beispielsweise in einer isotonischen Kochsalzlösung vor. Weitere Komponenten können beispielsweise Stabilisierungsmittel wie Tween 80 oder Pufferlösungen sein.

Zur Behandlung werden bevorzugt intakte trifunktionelle bispezifische und/oder trispezifische Antikörper eingesetzt. Durch die Behandlung wird nicht nur die oben beschriebene überraschende direkte Zerstörung der Tumorzellen erreicht, sondern auch eine gegen den zu behandelnden Tumor gerichtete Immunität induziert.

Unter "intakte Antikörper" sind solche zu verstehen, die einen funktionellen Fc-Teil besitzen. Bevorzugt handelt es sich hierbei um heterologe Antikörper, d.h. sie sind aus schweren Immunglobulinketten unterschiedlicher Subklassen (-kombinationen, auch Fragmente) und/oder Herkunft (Spezies) zusammengesetzt.

Neben den oben beschriebenen Merkmalen a, b und c weisen die eingesetzten Antikörper in bevorzugten Ausgestaltungen der Erfindung noch die weiteren Merkmale d und e auf:
d) aktiviert die Fc-Rezeptor positive Zelle durch seine Bindung an die Fc-Rezeptor positive Zelle, wodurch die Expression von Cytokinen und/oder von kostimulatorischen Antigenen initiiert oder erhöht wird;
e) die kostimulatorischen Antigene und/oder Cytokine übertragen an die T-Zelle zumindest ein 2. Aktivierungssignal, das für eine physiologische Aktivierung der T-Zelle benötigt wird, wobei sich diese Aktivierung in der Hochregulation von Aktivierungsmarkem, der Zerstörung der Tumorzelle und/oder in einer Proliferation der T-Zelle zeigt.

Die Erfindung wird nachfolgend insbesondere am Beispiel bispezifischer Antikörper beschrieben. Die Resultate sind aber auch mit trispezifischen Antikörpern erzielbar.

Die erfindungsgemäß verwendbaren Antikörper sind befähigt, die Fc-Rezeptor positive Zelle zu aktivieren, wodurch die Expression von Zytokinen und/oder kostimulatorischen Antigenen initiiert oder erhöht wird.

Bei den trispezifischen Antikörpern erfolgt die Bindung an die Fc-Rezeptor positiven Zellen bevorzugt beispielsweise über den Fc-Rezeptor von Fc-Rezeptor positiven Zellen oder auch über andere Antigene auf Fc-Rezeptor positiven Zellen (Antigenpräsentierenden Zellen), wie z.B. den Mannose-Rezeptor.

Durch die erfindungsgemäße Kombination und die Art der Anwendung der intakten, bevorzugt heterologen bispezifischen und/oder trispezifischen Antikörper wird im Patienten neben der Behandlung des Aszites und des Pleuraergusses durch direkte Zerstörung der Tumorzellen auch eine Tumorimmunität aufgebaut, bevorzugt eine Langzeit-Tumorimmunität. Die Verabreichung erfolgt bevorzugt in einen Patienten nach der Behandlung des Primärtumors, bevorzugt bei Patienten in einer Tumorresterkrankungssituation (MRD = minimal residual disease). Bei Patienten mit wenig verbliebenen Tumorzellen, bei denen allerdings die Gefahr eines Rezidivs hoch sein kann, ist die erfindungsgemäß beschriebene Anwendung der pharmazeutischen Zubereitung besonders erfolgreich.

Die erfindungsgemäß verwendbaren heterologen bispezifischen und/oder trispezifischen Antikörper sind an sich bekannt. Sie sind zum Beispiel beschrieben in Lindhofer et al., Blood, 88:4651, 1996 oder Lindhofer et al., J. Immunology, 155:219, 1995.

Auf der Tumorzelle erfolgt eine Hochregulation von MHC 1, sowie eine Aktivierung der intrazellulären Prozessierungsmaschinerie (Proteasom-Komplex) aufgrund der Freisetzung von Zytokinen (wie z.B. INF-γ und TNF-α) in unmittelbarer Nachbarschaft der Tumorzelle. Die Zytokine werden aufgrund bispezifischer Antikörper-vermittelter Aktivierung von T-Zellen und akzessorischen Zellen freigesetzt. D.h. durch den intakten bsAk werden nicht nur Tumorzellen zerstört oder phagozytiert, sondern indirekt auch deren Tumorimmunogenität erhöht.

Die Aktivierung der Fc-Rezeptor positiven Zelle durch den bsAk ist von der Subklasse bzw. der Subklassenkombination des bsAk abhängig. Wie in in vitro-Versuchen nachgewiesen werden konnte, sind beispielsweise bsAk der Subklassenkombination Maus-IgG2a/Ratte-IgG2b in der Lage, an Fc-Rezeptor positive Zellen zu binden und diese gleichzeitig zu aktivieren, was zur Hochregulation bzw. Neuausbildung (Expression) von kostimulatorischen Antigenen, wie z.B. CD40, CD80 oder CD86, auf der Zelloberfläche dieser Zellen führt (Zeidler et al., J. Immunol., 163:1246, 1999). Dagegen sind bsAk der Subklassenkombination Maus-IgG1/RatteIgG2b zwar in der Lage an Fc-Rezeptor positive Zellen zu binden (Haagen et al., J. Immunology, 1995, 154: 1852-1860), können diese aber offenbar nicht in vergleichbarem Maße aktivieren (Gast et al., Cancer Immunol. Immuntherap., 1995, 40: 390).

Während der intakte bsAk die T-Zelle mit einem Bindungsarm (z.B. an CD3 oder CD2) bindet und gleichzeitig aktiviert, können kostimulatorische Signale von der an den Fc-Teil des bsAk gebundenen Fc-Rezeptor positiven Zelle an die T-Zelle übermittelt werden. D.h. erst die Kombination von Aktivierung der T-Zelle über einen Bindungsarm des bsAk und der gleichzeitigen Übertragung von kostimulatorischen Signalen von der Fc-Rezeptor positiven Zelle auf die T-Zelle, führt zu einer effizienten T-Zellaktivierung. Tumorspezifische T-Zellen, die an der Tumorzelle ungenügend aktiviert wurden und anergisch sind, können nach der erfindungsgemäßen Behandlung mit intakten bispezifischen Antikörpern oder trispezifischen Antikörpern ebenfalls reaktiviert werden.

Ein weiterer wichtiger Aspekt bei der Induktion einer Tumorimmunität ist die mögliche Phagozytose, Prozessierung und Präsentation von Tumorbestandteilen durch die vom bsAk herangeführten und aktivierten akzessorischen Zellen (Monozyten/Makrophagen, oder dendritische Zellen). Durch diesen klassischen Mechanismus der Präsentation von Antigenen können sowohl tumorspezifische CD4- wie auch CD8-positive Zellen generiert werden. Tumorspezifische CD4-Zellen spielen darüber hinaus eine wichtige Rolle für die Induktion einer humoralen Immunantwort im Zusammenhang mit der T-B-Zell Kooperation.

Bispezifische und trispezifische Antikörper können mit einem Bindungsarm an den T-Zellrezeptor-Komplex der T-Zelle, mit dem zweiten Bindungsarm an tumorassoziierte Antigene auf der Tumorzelle binden. Sie aktivieren dabei T-Zellen, die durch Freisetzung von Zytokinen oder Apoptose-vermittelnde Mechanismen die Tumorzellen zerstören. Darüber hinaus besteht offenbar die Möglichkeit, daß T-Zellen im Rahmen der Aktivierung mit bispezifischen Antikörpern tumorspezifische Antigene über ihren Rezeptor erkennen und dadurch eine dauerhafte Immunisierung eingeleitet wird. Von besonderer Bedeutung ist dabei der intakte Fc-Teil des bispezifischen oder trispezifischen Antikörpers, der die Bindung an akzessorische Zellen wie z.B. Monozyten/Makrophagen und dendritische Zellen vermittelt und diese veranlaßt selbst zytotoxisch zu werden und/oder gleichzeitig wichtige kostimulatorische Signale an die T-Zelle weiterzugeben. Auf diese Weise kann offensichtlich eine T-Zellantwort u.U. auch gegen bislang unbekannte, tumorspezifische Peptide induziert werden.

Durch Redirektion von u. U. anergisierten, tumorspezifischen T-Zellen an Tumorzellen mittels bispezifischer und/oder trispezifischer Antikörper bei gleichzeitiger Kostimulation derartiger T-Zellen durch akzessorische Zellen welche an den Fc-Teil des bispezifischen oder trispezifische Antikörpers binden, könnte die Anergie von zytotoxischen T-Zellen (CTLs) aufgehoben werden. D.h. eine im Patienten gegen den Tumor existierende T-Zell-Toleranz kann mittels intakter heterologer bispezifischer und/oder trispezifischer Antikörper gebrochen und damit eine dauerhafte Tumorimmunität induziert werden.

Für den letzten Punkt gibt es erste in vivo-Daten aus Mausversuchen, die auf eine derartige dauerhafte Tumorimmunität nach Behandlung mit einem syngenen Tumor und intakten bsAk hinweisen. In diesen Versuchen überlebten insgesamt 14 von 18 Tieren, die nach einer ersten Tumorinjektion erfolgreich mit bsAk behandelt werden konnten, eine weitere Tumorinjektion 144 Tage nach der ersten Tumorinjektion - ohne eine erneute Gabe von bsAk.

Die erfindungsgemäß eingesetzten Antikörper sind bevorzugt zur Reaktivierung von in Anergie befindlichen, tumorspezifischen T-Zellen befähigt Weiterhin sind sie zur Induktion von tumorreaktiven Komplement-bindenden Antikörpern und damit zur Induktion einer humoralen Immunantwort in der Lage.

Die Bindung erfolgt bevorzugt über CD3, CD2, CD4, CD5, CD6, CD8, CD28, CD40L und/oder CD44 an die T-Zelle. Die Fc-Rezeptor positiven Zellen weisen zumindest einen Fcγ-Rezeptor Typ I oder III auf.

Erfindungsgemäß einsetzbare Antikörper sind zur Bindung an Monozyten, Makrophagen, dendritische Zellen, "Natural Killer"-Zellen (NK-Zellen) und/oder aktivierte neutrophile Zellen als Fcγ-Rezeptor I und/oder III positive Zellen befähigt (Zeidler, 1999, Zeidler 2000 a.a.o.).

Die erfindungsgemäß einsetzbaren Antikörper bewirken, daß die Expression von CD40, CD80, CD86, ICAM-1 und/oder LFA-3 als kostimulatorische Antigene oder/und die Sekretion von Zytokinen durch die Fc-Rezeptor positive Zelle initiiert oder erhöht wird. Die Zytokine sind bevorzugt IL-1, IL-2, IL-4, IL-6, IL-8,IL-12, INF-γ und/oder TNF-α.

Die erfindungsgemäß einsetzbaren bispezifischen Antikörper sind beispielsweise:
- ein anti-CD3 X anti-Tumor-assoziiertes Antigen- und/oder anti-CD4 X anti-Tumor-assoziiertes Antigen- und/oder anti-CD5 X anti-Tumor-assoziiertes Antigen- und/oder anti-CD6 X anti-Tumor-assoziiertes Antigen- und/oder anti-CD8 X anti-Tumor-assoziiertes Antigen- und/oder anti-CD2 X anti-Tumor-assoziiertes Antigen- und/oder anti-CD28 X anti-Tumor-assoziiertes Antigen- und/oder anti-CD40L X anti-Tumor-assoziiertes Antigen-Antikörper und/oder anti-CD44 X anti-Tumor-assoziiertes Antigen-Antikörper.

Die erfindungsgemäß einsetzbaren trispezifischen Antikörper sind bevorzugt:
- ein anti-CD3 X anti-Tumor-assoziiertes Antigen- und/oder anti-CD4 X anti-Tumor-assoziiertes Antigen- und/oder anti-CD5 X anti-Tumor-assoziiertes Antigen- und/oder anti-CD6 X anti-Tumor-assoziiertes Antigen- und/oder anti-CD8 X anti-Tumor-assoziiertes Antigen- und/oder anti-CD2 X anti-Tumor-assoziiertes Antigen- und/oder anti-CD28 X anti-Tumor-assoziiertes Antigen- und/oder anti-CD40L X anti-Tumor-assoziiertes Antigen-Antikörper und/oder anti-CD44 X anti-Tumor-assoziiertes Antigen-Antikörper.

Die erfindungsgemäß einsetzbaren trispezifischen Antikörper weisen zumindest eine T-Zell-Bindungsarm, einen Tumorzell-Bindungsarm und einen an Fc-Rezeptor positive Zellen bindenden Bindungsarm auf Dieser zuletzt genannte Bindungsarm kann ein anti-Fc-Rezeptor-Bindungsarm oder ein Mannose-Rezeptor-Bindungsarm sein.

Der bispezifische Antikörper ist bevorzugt ein heterologer intakter Ratte/Maus bispezifischer Antikörper.

Mit den erfindungsgemäß einsetzbaren bispezifischen und trispezifischen Antikörpern werden T-Zellen aktiviert und gegen die Tumorzellen redirigiert. Bevorzugt einsetzbare heterologe intakte bispezifische Antikörper werden aus einer oder mehreren der nachfolgenden Isotyp-Kombinationen ausgewählt:
Ratte-IgG2b/Maus-IgG2a,
   Ratte-IgG2b/Maus-IgG2b,
   Ratte-IgG2b/Maus-IgG3,
Ratte-IgG2b/Human-IgG1,
   Ratte-IgG2b/Human-IgG2,
   Ratte-IgG2b/Human-IgG3[orientaler Allotyp G3m(st) = Bindung an Protein A],
   Ratte-IgG2b/Human-IgG4,
Ratte-IgG2b/Ratte-IgG2c,
   Maus-IgG2a/Human-IgG3[kaukasische Allotypen G3m(b+g) = keine Bindung an Protein A, im folgenden mit * gekennzeichnet]
Maus-IgG2a/Maus-[VH-CH1,VL-CL]-Human-IgG1-[Hinge]-Human-IgG3*-[CH2-CH3]
Maus-IgG2a/Ratte-[VH-CH1, VL-CL]-Human-IgG1-[Hinge]-Human-IgG3*-[CH2-CH3]
Maus-IgG2a/Human-[VH-CH1,VL-CL]-Human-IgG1-[Hinge]-Human-IgG3*-[CH2-CH3]
Maus-[VH-CH1,VL-CL]-Human-IgG1/Ratte-[VH-CH1,VL-CL]-Human-IgG1-[Hinge]-Human-IgG3*-[CH2-CH3]
Maus-[VH-CH1,VL-CL]-Human-IgG4/Ratte-[VH-CH1,VL-CL]-Human-IgG4-[Hinge]-Human-IgG4[N-terminale Region von CH2]-Human-IgG3*[C-terminale Region von CH2: > AS-Position 251]-Human-IgG3*[CH3]
Ratte-IgG2b/Maus-[VH-CH1,VL-CL]-Human-IgG1-[Hinge-CH2-CH3]
Ratte-IgG2b/Maus-[VH-CH1,VL-CL]-Human-IgG2-[Hinge-CH2-CH3]
Ratte-IgG2b/Maus-[VH-CH1,VL-CL]-Human-IgG3-[Hinge-CH2-CH3, orientaler Allotyp]
Ratte-IgG2b/Maus-[VH-CH1,VL-CL]-Human-IgG4-[Hinge-CH2-CH3]
Human-IgG1/Human-[VH-CH1,VL-CL]-Human-IgG1-[Hinge]-Human-IgG3*-[CH2-CH3]
Human-IgG1/Ratte-[VH-CH1,VL-CL]-Human-IgG1-[Hinge]-Human-IgG4[Nterminale Region von CH2]-Human-IgG3*[C-terminale Region von CH2 : > AS-Position 251]-Human-IgG3*[CH3]
Human-IgG1/Maus-[VH-CH1,VL-CL]-Human-IgG1-[Hinge]-Human-IgG4[Nterminale Region von CH2]-Human-IgG3*[C-terminale Region von CH2 : > AS-Position 251]-Human-IgG3*[CH3]
Human-IgG1/Ratte-[VH-CH1,VL-CL]-Human-IgG1-[Hinge]-Human-IgG2[Nterminale Region von CH2]-Human-IgG3*[C-terminale Region von CH2 : > AS-Position 251]-Human-IgG3*[CH3]
Human-IgG1/Maus-[VH-CH1,VL-CL]-Human-IgG1-[Hinge]-Human-IgG2[N-terminale Region von CH2]-Human-IgG3*[C-terminale Region von CH2 : > AS-Position 251]-Human-IgG3*[CH3]
Human-IgG1/Ratte-[VH-CH1,VL-CL]-Human-IgG1-[Hinge]-Human-IgG3*-[CH2-CH3]
Human-IgG1/Maus-[VH-CH1,VL-CL]-Human-IgG1-[Hinge]-Human-IgG3*-[CH2-CH3]
Human-IgG2/Human-[VH-CH1,VL-CL]-Human-IgG2-[Hinge]-Human-IgG3*-[CH2-CH3]
Human-IgG4/Human-[VH-CH1,VL-CL]-Human-IgG4-[Hinge]-Human-IgG3*-[CH2-CH3]
Human-IgG4/Human-[VH-CH1,VL-CL]-Human-IgG4-[Hinge]-Human-IgG4[Nterminale Region von CH2]-Human-IgG3*[C-terminale Region von CH2 : > AS-Position 251]-Human-IgG3*[CH3]
Maus-IgG2b/Ratte-[VH-CH1,VL-CL]-Human-IgG1-[Hinge]-Human-IgG3*-[CH2-CH3]
Maus-IgG2b/Human-[VH-CH1,VL-CL]-Human-IgG1-[Hinge]-Human-IgG3*-[CH2-CH3]
Maus-IgG2b/Maus-[VH-CH1,VL-CL]-Human-IgG1-[Hinge]-Human-IgG3*-[CH2-CH3]
Maus-[VH-CH1,VL-CL]-Human-IgG4/Ratte-[VH-CH1,VL-CL]-Human-IgG4-[Hinge]-Human-IgG4-[CH2]-Human-IgG3*-[CH3]
HumanIgG1/Ratte[VH-CH1,VL-CL]-Human-IgG1[Hinge]-Human-IgG4-[CH2]-HumanIgG3*[CH3]
HumanIgG1/Maus[VH-CH1,VL-CL]-Human-IgG1[Hinge]-Human-IgG4-[CH2]-Human-IgG3*[CH3]
Human-IgG4/Human[VH-CH1,VL-CL]-Human-IgG4-[Hinge]-Human-IgG4-[CH2]-HumanIgG3*-[CH3]

Bei den erfindungsgemäß verwendbaren Antikörpern handelt es sich vorzugsweise um monoklonale, chimäre, rekombinante, synthetische, halbsynthetische oder chemisch modifizierte intakte Antikörper mit beispielsweise Fv-, Fab-, scFv- oder F(ab)₂-Fragmenten.

Bevorzugt werden Antikörper oder Derivate oder Fragmente vom Menschen verwendet oder solche, die derart verändert sind, daß sie sich für die Anwendung beim Menschen eignen (sogenannte "humanized antibodies") (siehe z.B. Shalaby et al., J. Exp. Med. 175 (1992), 217; Mocikat et al., Transplantation 57 (1994), 405).

Die Herstellung der verschiedenen oben genannten Antikörpertypen und -fragmente ist dem Fachmann geläufig. Die Herstellung monoklonaler Antikörper, die ihren Ursprung vorzugsweise in Säugetieren, z.B. Mensch, Ratte, Maus, Kaninchen oder Ziege, haben, kann mittels herkömmlicher Methoden erfolgen, wie sie z.B. in Köhler und Milstein (Nature 256 (1975), 495), in Harlow und Lane (Antibodies, A Laboratory Manual (1988), Cold Spring Harbor) oder in Galfré (Meth. Enzymol. 73 (1981), 3) oder der DE 195 31 346 beschrieben sind.

Ferner ist es möglich, die beschriebenen Antikörper mittels rekombinanter DNA-Technologie nach dem Fachmann geläufigen Techniken herzustellen (siehe Kurucz et al., J. Immunol. 154 (1995), 4576; Holliger et al., Proc. Natl. Acad. Sc. USA 90 (1993), 6444).

Die Herstellung von Antikörpern mit zwei verschiedenen Spezifitäten, den sogenannten bispezifischen Antikörpern, ist zum einen durch Anwendung rekombinanter DNA-Technologie möglich, aber auch durch die sogenannte Hybrid-Hybridoma-Fusionstechnik (siehe z.B. Milstein et al., Nature 305 (1983), 537). Hierbei werden Hybridomazellinien, die Antikörper mit jeweils einer der gewünschten Spezifitäten produzieren, fusioniert und Zellklone (Quadrome) identifiziert und isoliert, die Antikörper mit beiden Spezifitäten produzieren.

Das der Erfindung zugrunde liegende Problem kann sowohl durch bispezifische als auch trispezifische Antikörper gelöst werden, die bevorzugt die gekennzeichneten Eigenschaften und Wirkungen aufweisen. Nachfolgend wird die Herstellung von Antikörpern mit zwei und drei Spezifitäten näher beschrieben. Die Bereitstellung derartiger bispezifischer und trispezifischer Antikörper gehört zum Stand der Technik, und auf die derartige Herstellungstechniken beschreibende Literatur wird hier voll inhaltlich Bezug genommen.

Die Herstellung von Antikörpern mit drei Spezifitäten, sogenannten trispezifischen Antikörpern, durch die das der Erfindung zugrundeliegende Problem ebenfalls lösbar ist, kann beispielsweise derart erfolgen, daß an eine der schweren Ig-Ketten eines bispezifischen Antikörpers eine dritte Antigenbindungsstelle mit einer weiteren Spezifität, z.B. in Form eines "single chain variable fragments" (scFv), angekoppelt wird. Das scFv kann beispielsweise über einen

-S-S(G₄S)ₙD-I-Linker

an eine der schweren Ketten gebunden sein (S = Serin, G = Glycin, D = Aspartat, I = Isoleucin).

Analog dazu können trispezifische F(ab)₂-Konstrukte hergestellt werden, indem die CH2-CH3-Regionen der schweren Kette einer Spezifität eines bispezifischen Antikörpers ersetzt werden durch ein scFv mit einer dritten Spezifität, während die CH2-CH3-Regionen der schweren Kette der anderen Spezifität beispielsweise durch Einbau eines Stopcodons (am Ende der "Hinge"-Region) in das codierende Gen, z.B. mittels homologer Rekombination, entfernt werden.

Möglich ist auch die Herstellung trispezifischer scFv-Konstrukte. Hierbei werden drei VH-VL-Regionen, die drei verschiedene Spezifitäten repräsentieren, hintereinander in Reihe angeordnet.

Erfindungsgemäß werden z.B. intakte bispezifische Antikörper verwendet. Intakte bispezifische Antikörper sind aus zwei Antikörper-Halbmolekülen (je eine H- und L-Immunglobulinkette), die jeweils eine Spezifität repräsentieren, zusammengesetzt, und besitzen darüber hinaus, wie normale Antikörper auch, einen Fc-Teil mit den bekannten Effektorfunktionen. Sie werden bevorzugt durch die Quadrom-Technologie hergestellt. Dieses Herstellungsverfahren ist beispielhaft in der DE-A-44 19 399 beschrieben. Auf diese Druckschrift, auch bzgl. einer Definition der bispezifischen Antikörper, wird zur vollständigen Offenbarung vollinhaltlich Bezug genommen. Selbstverständlich sind auch andere Herstellungsverfahren einsetzbar, solange sie zu den erfindungsgemäß notwendigen intakten bispezifischen Antikörpern der obigen Definition führen.

Beispielsweise können in einem neu entwickelten Herstellungsverfahren (Lindhofer et al., J. Immunology 1995, 155: 219) intakte bispezifische Antikörper in ausreichender Menge produziert werden. Die Kombination von 2 bispezifischen Antikörpern gegen 2 unterschiedliche tumorassoziierte Antigene (z.B. c-erb-B2, und EpCAM) auf den Mammakarzinomzellen minimiert die Gefahr, daß Tumorzellen, die nur ein Antigen exprimieren, unerkannt bleiben.

Weitere Vorteile von intakten bsAk mit der Fähigkeit zur Redirektion von T-Zellen gegenüber den o.g. bsF(ab')2 Fragmenten sind im einzelnen:
1. An intakte bsAk können Fc-Rezeptor positive Zellen binden und einerseits über ADCC (antibody-dependent cell-mediated cytotoxicity) direkt zur Tumorzerstörung beitragen sowie andererseits wie oben näher ausgeführt zur T-Zellaktivierung.
2. Durch intakte T-Zell-redirigierende bsAk werden auch anergisierte tumorspezifische T-Zellen an die Tumorzelle herangeführt, die erfindungsgemäß direkt am Tumor reaktiviert werden können. Dies kann durch ein bsF(ab')2-Fragment mit den Spezifitäten anti-CD64Xanti-tumorassoziiertes Antigen nicht erreicht werden.

Die Bindung des bsAk an Fcγ-RI besitzt zwei wesentliche Vorteile im Hinblick auf eine optimale anti-Tumorwirksamkeit:
(1) Fcγ-RI positive Zellen besitzen die Fähigkeit mittels ADCC Tumorzellen zu eliminieren und können insofern synergistisch zur anti-Tumorwirkung der durch den bsAk an die Tumorzelle herangeführten cytotoxischen T-Zellen beitragen.
(2) Fcγ-RI positive Zellen (wie z.B. Monozyten/Makrophagen/Dendriten) sind in der Lage, wichtige kostimulatorische Signale, ähnlich wie bei der Antigen-Präsentation, der T-Zelle zu liefern und damit eine Anergisierung der T-Zelle zu verhindern. Es können weiterhin, als erwünschtes Nebenprodukt, aufgrund der durch intakten bsAk vermittelten Interaktion von T-Zelle mit akzessorischer Zelle und Tumorzelle sogar T-Zellen, deren T-Zellrezeptor tumorspezifische Peptide (über MHC Antigene auf der Tumorzelle präsentiert) erkennt, stimuliert werden. Die für eine korrekte Aktivierung der T-Zelle notwendigen Kostimuli würden in dieser Konstellation von der akzessorischen Zelle (z.B. Monocyt) geliefert werden. Insofern sollte der erfindungsgemäße Antikörper neben der direkten T-Zellrezeptor-unabhängigen, durch bsAk vermittelten Tumorzerstörung ebenfalls tumorspezifische T-Zellen aktivieren und generieren, die nach Abbau der bsAk weiterhin im Patienten patrouillieren. D.h. mittels intakter bsAk kann ähnlich wie bei gentherapeutischen Ansätzen (z.B. durch Einbau von kostimulatorischen Antigenen wie B-7 in die Tumorzelle) die Tumortoleranz im Patienten durchbrochen werden.

Wie überraschend in zwei Patienten gezeigt werden konnte, kann die Immuntherapie von Tumoren mittels trifunktioneller bispezifischer Antikörper auch bei der Behandlung von Aszites therapeutisch eingesetzt werden.

Bei einer Patientin mit Ovarialkarzinom konnte die Neubildung von Aszites nach einer Behandlung mit bsAk für einen Zeitraum von 6 Monaten unterbunden werden. Dabei konnte auch die vollständige Zerstörung der Tumorzellen, die in der Aszitesflüssigkeit vorhanden sind, nachgewiesen werden.

### Beispiel 1

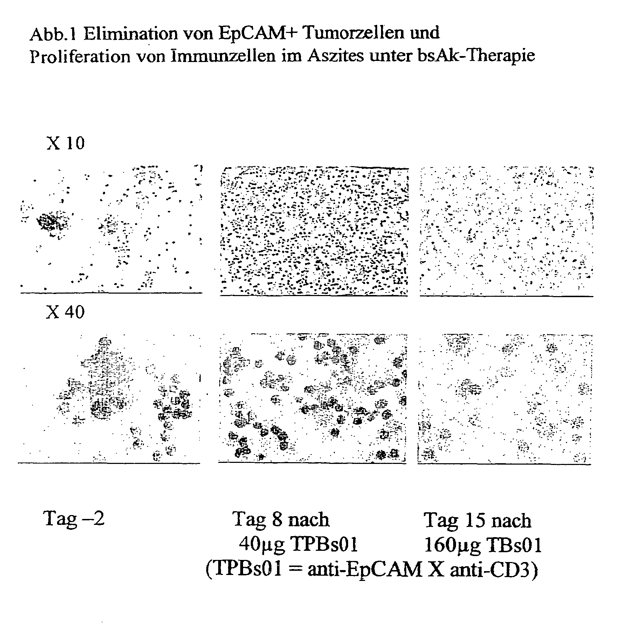

Bei einer Patientin mit Ovarialkarzinom wurde zunächst das Peritoneum punktiert, Aszitesflüssigkeit (700ml) entnommen und die darin enthaltenen Zellen analysiert. Wie in Abb. 1 gezeigt, konnten die Tumorzellen bereits visuell mittels Mikroskop auf Grund ihrer Morphologie eindeutig identifiziert werden. Zusätzlich wurde eine anti-EpCAM Peroxidasefärbung durchgeführt.

Die Auszählung der Zellen im Aszites vor der Behandlung ergab ein Verhältnis von ca. 50:50 von Tumorzellen und Immunzellen.

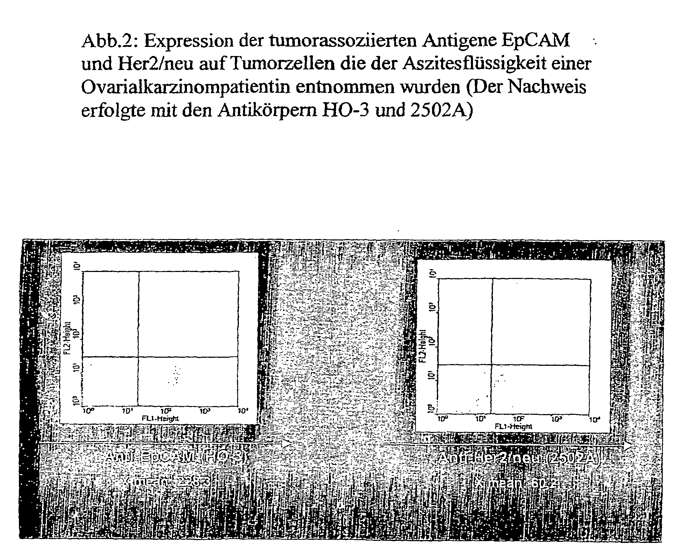

Die durchflußzytometrische Analyse ergab eine starke Positivität der Tumorzellen für das Oberflächenantigen EpCAM (epithelial cell adhesion molecule, Balzar et al., Mol. Cell. Biol., 18:4833, 1998) sowie eine schwächere Expression des tumorassoziierten Oberflächenantigens Her2/neu (Abb. 2).

Um die Möglichkeit einer Fremdproteinreaktion auszuschließen wurden zunächst 10µg des bsAk TPBS03 REXOMAB (anti-Her2/neu X anti-CD3; Isotypkombination MausIgG2a x RatteIgG2b) intraperitoneal mittels Perfuorspritze über 8h infundiert. Die genaue Applikation ist in Tabelle 1 dargestellt.

**Tabelle 1:**

| Dosierungsschema | | |
|---|---|---|
| Tag | Bispezifischer Antikörper | Dosis |
| 1 | CD3xHer2/neu | 10 µg |
| 4 | CD3xEpCAM | 10 µg |
| 7 | CD3xEpCAM | 40 µg |
| 9 | CD3xEpCAM | 80 µg |
| 11 | CD3xEpCAM | 160 µg |
| 15 | CD3xEpCAM + CD3xHer2/neu | 200 µg + 50 µg |

Eine erste Veränderung konnte 1 Tag nach der 40µg Infusion festgestellt werden. Es wurde zu diesem Zeitpunkt eine Peritoneallavage durchgeführt und die darin enthaltenen Zellen analysiert, wobei sich das Verhältnis von Immunzellen zu Tumorzellen auf den Wert 3:1 verbessert hatte.

Überraschenderweise konnten am Tag 11 unmittelbar vor der 160µg Infusion bei der zweiten Peritoneallavage nur noch vereinzelte Tumorzellen gefunden werden. Das Verhältnis von Immunzellen zu Tumorzellen hatte sich dramatisch auf den Wert 330:1 verändert.

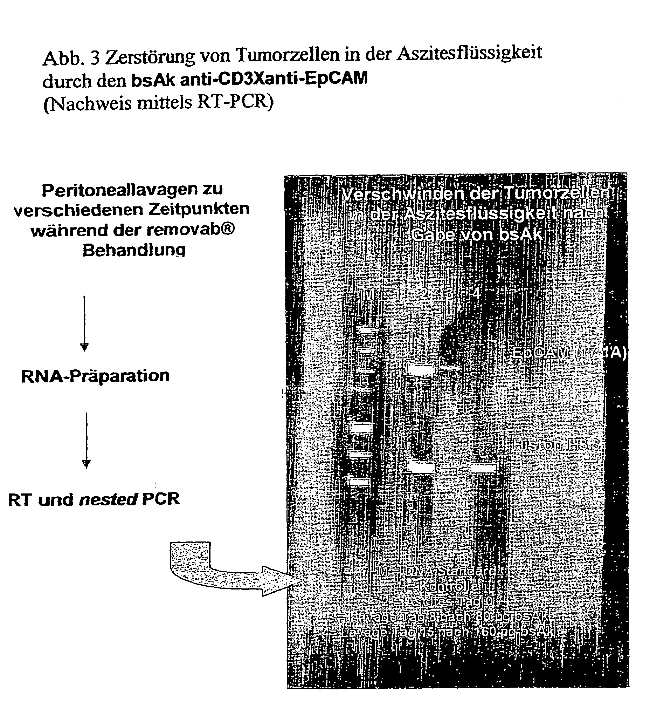

In der dritten Peritoneallavage am Tag 15 nach Gabe von 160µg und unmittelbar vor der 250µg Infusion konnten keinerlei Tumorzellen mehr nachgewiesen werden (Abb. 1). Dieses Ergebnis konnte mittels einer besonders sensitiven nested-EpCAM RT-PCR bestätigt werden (Abb.3).

Es ist festzuhalten, dass es während der Behandlung zu keinerlei schwereren Nebenwirkungen kam. Lediglich nach der höchsten Dosis von 250µg bsAk kam es kurzzeitig zu Fieber (38,6°C).

Bei der Patientin konnte über einen Zeitraum von 6 Monaten keine Neubildung der Aszitesflüssigkeit beobachtet werden und es musste auch keine weitere Punktion durchgeführt werden. Dies ist insofern bemerkenswert da das Krankheitsstadium bei dieser Patientin zum Zeitpunkt des Beginns der Immuntherapie sehr fortgeschritten war und bereits Lebermetastasen existierten.

### Beispiel 2

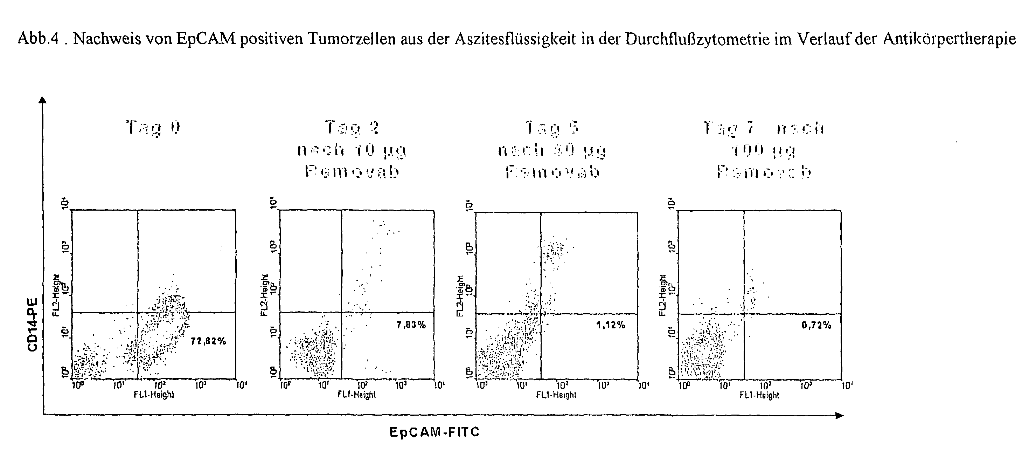

Bei einer weiteren Patientin mit Mammakarzinom und Aszitesbildung wurde ebenfalls das Peritoneum punktiert, die Aszitesflüssigkeit (3 Liter) gesammelt und die darin enthaltenen Zellen in der Durchflusszytometrie analysiert. Es wurden dabei Nachweisantikörper gegen das tumorassoziiert Antigen EpCAM und CD14 verwendet um eine unspezifische Bindung an Fc-Rezeptor positive Zellen (der Nachweisantikörper) von einer Bindung an Tumorzellen unterscheiden zu können. Wie in Abb. 4 dargestellt, waren zum Zeitpunkt Tag 0 vor der Therapie, über 70% der in der Aszitesflüssigkeit nachweisbaren Zellen, EpCAM-positive Tumorzellen die allerdings unter der Therapie auf 0,7% abnahmen.

Die Patientin erhielt am Tag 0, nach Ablassen der Aszitesflüssigkeit, 10µg des anti-EpCAM X anti-CD3 Antikörpers Removab (Isotypkombination: Maus IgG2a x RatteIgG2b), sowie an den darauffolgenden Tagen 2 und 5 jeweils 40µg und 100µg Removab intraperitoneal über einen Zeitraum von ca. 6 Stunden verabreicht. Am Tag 8 wurde der Patientin eine Kombination der Antikörper Rexomab (anti-Her2/neu X anti-CD3; 100µg) und Removab (50µg) intraperitoneal appliziert, nachdem in der Durchflußzytometrie ebenfalls das tumorassoziierte Antigen Her2/neu auf den Tumorzellen nachgewiesen werden konnte (nicht gezeigt).

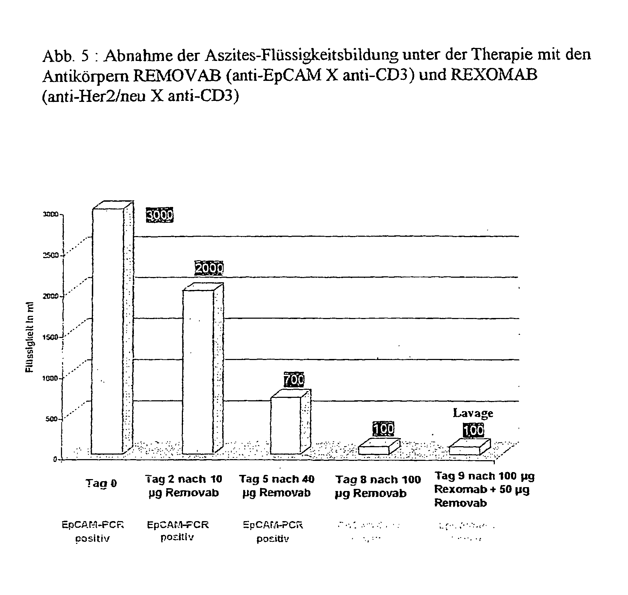

Wie in Abb.5 dargestellt, nahm unter der Therapie die Flüssigkeitsneubildung des Aszites kontinuierlich ab und blieb zum Ende der Therapie ganz aus.

Die Behandlung wurde gut vertragen. Es kam allerdings nach der höchsten Dosis zu einem vorübergehendem Anstieg der Leberwerte und kurzzeitig zu Fieber.

### Schlussfolgerung:

Aus diesen Ergebnissen lässt sich schließen, dass bereits eine Dosis von 40µg bsAk eine gewisse anti-Tumor Wirksamkeit besitzt und es zur Aktivierung und Proliferation von Immun-Effektorzellen kommt. Nach Gabe von 160µg bsAk konnte selbst mit sehr sensitiven Nachweismethoden kein Hinweis auf das Vorhandensein von restlichen Tumorzellen in der Peritoneallavage der ersten Patientin mehr gefunden werden und somit von einer effektiven Zerstörung der autologen Tumorzellen in der Aszitesflüssigkeit ausgegangen werden. Ein ähnlicher Effekt konnte auch bei der zweiten Patientin nach Gabe von 100 µg REMOVAB festgestellt werden. Die Verträglichkeit der verabreichten Dosen war gut, es kam es lediglich bei einer der Patientinnen zu einer Erhöhung der Leberwerte oder sonstigen negativen Nebenwirkungen. Nach der 250µg Dosis (Patientin 1) bzw. 100 µg Dosis (Patientin 2) kam es kurzzeitig zu Fieber (38,6°C).

Insofern ließe sich für zukünftige Behandlungen zur Zerstörung von Tumorzellen im Aszites und der Vermeidung der Neubildung von Aszites folgende Applikation von trifunktionellen bispezifischen Antikörpern ableiten:
Tag 0 : 10µg (Einstiegsdosis zur Prüfung einer Überempfindlichkeitsreaktion gegen das Fremdprotein)
Tag 2 : 40µg (Aktivierung und Proliferation der Immunzellen)
Tag 4 : 200µg (Hauptdosis zur Zerstörung der Tumorzellen)

Nachdem es gegenwärtig keine befriedigende Behandlung von malignem Aszites bei Tumorerkrankungen gibt, stellt die Immuntherapie mittels bsAk hier eine neue, vielversprechende Methode dar.

Die immuntherapeutische Behandlung von Aszites intraperitoneal mittels trifunktioneller bsAk kann auch als Primärimmunisierung zur Induktion einer langanhaltenden anti-Tumor Immunität dienen.

Der Bauchraum enthält eine ganze Reihe von immunologischen Organen wie z.B. Milz, Peyer'sche Plaques und eine Vielzahl von Lymphknoten. Die trifunktionellen bsAk sind in der Lage auf Grund ihrer Interaktion mit Tumorzellen, T-Zellen und akzessorischen Zellen (Zeidler et al., J.Immunol. 163:1246, 1999, Zeidler et al., British J. Cancer 83:261, 2000), Tumormaterial dem antigenpräsentierendem System zuzuführen und eine Einwanderung von aktivierten dendritischen Zellen in die immunologischen Organe einzuleiten.

Diese Vorgänge sind essentielle Voraussetzungen für die Induktion einer Immunantwort gegen den autologen Tumor. Um die antitumorale Immunantwort langfristig zu etablieren und insbesondere eine funktionale polyklonale humorale und zelluläre Immunantwort zu generieren, ist eine Sekundärimmunisierung notwendig. Die Sekundärimmunisierung kann auch subcutan bzw. intradermal durchgeführt werden. Als autologes Tumormaterial kann bei malignem Aszites die in ihm befindlichen Tumorzellen einfach mittels Punktion gewonnen und verwendet werden.

## Patentansprüche

1. Verwendung eines trifunktionellen bispezifischen und/oder trispezifischen Antikörpers mit den nachfolgenden Eigenschaften:
a) bindet an eine T-Zelle;
b) bindet an zumindest ein Antigen auf einer Tumorzelle, die mit malignem Aszites und/oder einem Pleuraerguß in Zusammenhang steht;
c) bindet durch seinen Fc-Teil (bei bispezifischen Antikörpern) oder durch eine dritte Spezifität (bei trispezifischen Antikörpern) an Fc-Rezeptor positive Zellen
zur Herstellung einer pharmazeutischen Zusammensetzung zur Behandlung von malignem Aszites und/oder einem Pleuraerguß, zur Zerstörung der Tumorzellen in der Aszitesflüssigkeit und/oder im Pleuraerguß.

2. Verwendung nach Anspruch 1,
**dadurch gekennzeichnet, daß**
die pharmazeutische Zubereitung zur Verabreichung in Form einer ersten Dosis und zumindest einer weiteren Dosis ausgelegt ist.

3. Verwendung nach Anspruch 1 oder 2,
**dadurch gekennzeichnet, daß**
die pharmazeutische Zubereitung zur Verabreichung in Form einer zweiten Dosis zur Aktivierung und Proliferation der Immunzellen und zumindest einer dritten Dosis zur Zerstörung der Tumorzellen ausgelegt ist.

4. Verwendung nach einem oder mehreren der vorhergehenden Ansprüche,
**dadurch gekennzeichnet, daß**
die pharmazeutische Zubereitung zur Verabreichung in Form einer ersten Dosis zur Prüfung allergischer Reaktionen gegen den verabreichten Antikörper ausgelegt ist.

5. Verwendung nach einem oder mehreren der vorhergehenden Ansprüche,
**dadurch gekennzeichnet, daß**
die pharmazeutische Zubereitung so ausgelegt ist, daß zur Verabreichung am ersten Tag eine erste Dosis von 1 bis 20 µg, bevorzugt 5 bis 10 µg, des Antikörpers, zur Verabreichung der zweiten Dosis eine Menge von 20 bis 100 µg, bevorzugt 30 bis 60 µg, des Antikörpers, und zur Verabreichung jeder weiteren Dosis eine Menge von 100 bis 500 µg, bevorzugt 100 bis 300 µg, des Antikörpers enthalten ist

6. Verwendung nach einem oder mehreren der vorhergehenden Ansprüche,
**dadurch gekennzeichnet, daß**
der Antikörper in 3 bis 8 Dosen, bevorzugt 3 bis 6 Dosen, zur Primärimmunisierung verabreicht wird, bevorzugt zwischen jeder Applikation ein Zeitraum von ca. 2 bis 3 Tagen liegt, und weiterhin bevorzugt die Verabreichung des Antikörpers so lange erfolgen kann, bis eine Antikörperreaktion gegen den verabreichten Antikörper nachweisbar ist

7. Verwendung nach einem oder mehreren der vorhergehenden Ansprüche,
**dadurch gekennzeichnet, daß**
die pharmazeutische Zubereitung so ausgelegt ist, daß zur Sekundärimmunisierung bei der Verabreichung weiterer Dosen inaktivierte autologe und/oder allogene Tumorzellen und autologe Immunzellen enthalten sind.

8. Verwendung nach Anspruch 7,
**dadurch gekennzeichnet, daß**
als autologe Immunzellen Apheresezellen eingesetzt werden, die bevorzugt vor der ersten Chemotherapie gewonnen wurden.

9. Verwendung nach einem oder mehreren der vorhergehenden Ansprüche,
**dadurch gekennzeichnet, daß**
die pharmazeutische Zubereitung zur Primärimmunisierung bevorzugt für eine intraperitoneale Applikation ausgelegt ist und zur Sekundärimmunisierung für eine intradermale, subkutane oder intramuskuläre Applikation ausgelegt ist.

10. Verwendung nach Anspruch 7, 8 oder 9,
**dadurch gekennzeichnet, daß**
die pharmazeutische Zubereitung zur Sckundärimmunisierung die nachfolgenden Mengen enthält:
a) 1-200x10⁶ inaktivierte Tumorzellen, bevorzugt 10-100x10⁶ inaktivierte Tumorzellen
b) 10-500x10⁶ Immunzellen, bevorzugt 50-200x10⁶ Immunzellen
c) 1-10 µg trifunktionelle bispezifische Antikörper oder trispezifische Antikörper,
bevorzugt 2-5 µg Antikörper

11. Verwendung nach einem oder mehreren der vorhergehenden Ansprüche,
**dadurch gekennzeichnet, daß**
ein intakter trifunktioneller bispezifischer oder trispezifischer Antikörper eingesetzt wird.

12. Verwendung nach einem oder mehreren der vorhergehenden Ansprüche,
**dadurch gekennzeichnet, daß**
der Antikörper zur Bindung Fc-Rezeptor positiver Zellen befähigt ist, die einen Fcγ-Rezeptor I oder III aufweisen.

13. Verwendung nach einem oder mehreren der vorhergehenden Ansprüche,
**dadurch gekennzeichnet, daß**
die Antikörper zur Bindung an Monozyten, Makrophagen, dendritische Zellen, "Natural Killer"-Zellen (NK-Zellen) und/oder aktivierte neutrophile Zellen als Fcγ-Rezeptor I und/oder III positive Zellen befähigt sind.

14. Verwendung nach einem oder mehreren der vorhergehenden Ansprüche,
**dadurch gekennzeichnet, daß**
die Antikörper zur Induktion von tumorreaktiven Komplement-bindenden Antikörpern und damit zur Induktion einer humoralen Immunantwort befähigt sind.

15. Verwendung nach einem oder mehreren der vorhergehenden Ansprüche,
**dadurch gekennzeichnet, daß**
die Antikörper so ausgewählt werden, daß sie über CD2, CD3, CD4, CD5, CD6, CD8, CD28, CD40L (= CD154) und/oder CD44 an die T-Zellen binden.

16. Verwendung nach einem oder mehreren der vorhergehenden Ansprüche,
**dadurch gekennzeichnet, daß**
die Antikörper so ausgewählt werden, daß nach ihrer Bindung an die Fc-Rezeptor positiven Zellen die Expression von CD40, CD80, CD86, ICAM-1 und/oder LFA-3 als kostimulatorische Antigene und/oder die Sekretion von Zytokinen durch die Fc-Rezeptor positive Zelle initiiert oder erhöht wird

17. Verwendung nach einem oder mehreren der vorhergehenden Ansprüche,
**dadurch gekennzeichnet, daß**
die Antikörper so ausgewählt werden, daß die Sekretion der Zytokine IL-1, IL-2, IL-4, IL-6, IL-8, IL-12, INF-γ und/oder TNF-α erh öht wird.

18. Verwendung nach einem oder mehreren der vorhergehenden Ansprüche,
**dadurch gekennzeichnet, daß**
der bispezifische Antikörper so ausgewählt wird, daß er ein anti-CD3 X anti-Tumor-assoziiertes Antigen- und/oder anti-CD4 X anti-Tumor-assoziiertes Antigen- und/oder anti-CD5 X anti-Tumor-assoziiertes Antigen- und/oder anti-CD6 X anti-Tumor-assoziiertes Antigen- und/oder anti-CD8 X anti-Tumor-assoziiertes Antigen- und/oder anti-CD2 X anti-Tumor-assoziiertes Antigen- und/oder anti-CD28 X anti-Tumor-assoziiertes Antigen- und/oder anti-CD40L X anti-Tumor-assoziiertes Antigen- und/oder anti-CD44 X anti-Tumor-assoziiertes Antigen-Antikörper ist.

19. Verwendung nach einem oder mehreren der vorhergehenden Ansprüche,
**dadurch gekennzeichnet, daß**
der bispezifische Antikörper aus einem heterologen bispezifischen oder trispezifischen Antikörper, bevorzugt einem heterologen Ratte/Maus bispezifischen Antikörper, ausgewählt wird.

20. Verwendung nach einem oder mehreren der vorhergehenden Ansprüche,
**dadurch gekennzeichnet, daß**
der trispezifische Antikörper einen T-Zell-Bindungsarm, einen Tumorzell-Bindungsarm und eine dritte Spezifität zur Bindung an Fc-Rezeptor positive Zellen besitzt

21. Verwendung nach einem oder mehreren der vorhergehenden Ansprüche,
**dadurch gekennzeichnet, daß**
der trispezifische Antikörper so ausgewählt wird, daß er ein anti-CD3 X anti-Tumor-assoziiertes Antigen- und/oder anti-CD4 X anti-Tumor-assoziiertes Antigen- und/oder anti-CD5 X anti-Tumor-assoziiertes Antigen- und/oder anti-CD6 X anti-Tumor-assoziiertes Antigen- und/oder anti-CD8 X anti-Tumor-assoziiertes Antigen- und/oder anti-CD2 X anti-Tumor-assoziiertes Antigen- und/oder anti-CD28 X anti-Tumor-assoziiertes Antigen- und/oder anti-CD40L X anti-Tumor-assoziiertes Antigen- und/oder anti-CD44 X anti-Tumor-assoziiertes Antigen-Antikörper ist.

22. Verwendung nach einem oder mehreren der vorhergehenden Ansprüche, 7, 8 und 10
**dadurch gekennzeichnet, daß**
Tumorzellen eingesetzt werden, die durch Bestrahlung, bevorzugt durch γ-Bestrahlung, weiterhin bevorzugt in einer Dosisstärke von 50 bis 200 Gy, oder durch chemische Substanzen, bevorzugt Mitomycin C, behandelt wurden.

23. Verwendung nach einem oder mehreren der vorhergehenden Ansprüche, 7, 8 und 10
**dadurch gekennzeichnet, daß**
zur Steigerung der Immunogenität der Tumorzellen diese vor Verabreichung einer Hitzevorbehandlung unterzogen werden.

24. Verwendung nach einem oder mehreren der vorhergehenden Ansprüche,
**dadurch gekennzeichnet, daß**
der Antikörper weiterhin so ausgewählt wird, daß er zur Aktivierung Fc-Rezeptor positiver Zellen befähigt ist, wodurch die Expression von Cytokinen und/oder costimulatorischen Antigenen initiiert oder erhöht wird.

25. Verwendung nach einem oder meheren der vorhergehenden Ansprüche,
**dadurch gekennzeichnet, daß**
autologe Immunzellen aus PBMC, Apherisaten oder Knochenmark eingesetzt werden.

26. Verwendung nach einem oder mehreren der vorhergehenden Ansprüche,
**dadurch gekennzeichnet, daß**
der bispezifische und/oder trispezifische Antikörper in einer Menge von 0,1 - 500 µg, die autologen oder allogenen Tumorzellen in einer Menge von 10⁵-2 x 10⁸ und die autologen Immunzellen in einer Menge von 5 x 10⁶-5 x 10⁸ Zellen vorliegen.

27. Verwendung nach einem oder mehreren der vorhergehenden Ansprüche,
**dadurch gekennzeichnet, daß**
zusätzlich zur Zerstörung der Tumorzellen eine Immunität gegen die Tumorzellen, gegen den die trifunktionellen bispezifischen Antikörper oder trispezifischen Antikörper gerichtet sind, etabliert wird.

## Claims

1. The use of a trifunctional bispecific and/or trispecific antibody having the following properties of:
a) binding to a T cell;
b) binding to at least one antigen on a tumor cell associated with malignant ascites and/or pleural effusion;
c) binding, by its Fc portion (in the case of bispecific antibodies) or by a third specificity (in the case of trispecific antibodies), to Fc receptor-positive cells
for the preparation of a pharmaceutical composition for the treatment of malignant ascites and/or pleural effusion for the destruction of tumor cells in the ascites fluid and/or in the pleural effusion.

2. The use according to claim 1
**characterized in that**
said pharmaceutical preparation is adapted for the administration in the form of a first dose and of at least one further dose.

3. The use according to claim 1 or 2,
**characterized in that**
said pharmaceutical preparation is adapted for the administration in the form of a second dose for the activation and proliferation of the immune cells and of at least a third dose for the destruction of the tumor cells.

4. The use according to one or more of the preceding claims,
**characterized in that**
said pharmaceutical preparation is adapted for the administration in the form of a first dose to test for allergic reactions against the antibody administered.

5. The use according to one or more of the preceding claims,
**characterized in that**
said pharmaceutical preparation is adapted to contain for the administration on the first day a first dose of 1 to 20 µg, preferably 5 to 10 µg of the antibody, for the administration of the second dose an amount of 20 to 100 µg, preferably 30 to 60 µg of the antibody, and for the administration of any further dose an amount of 100 to 500 µg, preferably 100 to 300 µg of the antibody.

6. The use according to one or more of the preceding claims,
**characterized in that**
said antibody is administered for primary immunization in 3 to 8 doses, preferably 3 to 6 doses, **in that** preferably an interval of about 2 to 3 days lies between each application, and **in that** further preferred the administration of the antibody can be performed for a sufficient time that an antibody reaction against the antibody administered can be detected.

7. The use according to one or more of the preceding claims,
**characterized in that**
for secondary immunization during the administration of further doses said pharmaceutical preparation is adapted to contain inactivated autologous and/or allogeneic tumor cells and autologous immune cells.

8. The use according to claim 7
**characterized in that**
apheresis cells preferably obtained prior to the first chemotherapy are used as said autologous immune cells.

9. The use according to one or more of the preceding claims,
**characterized in that**
said pharmaceutical preparation preferably is adapted for an intraperitoneal application for primary immunization and is adapted for an intradermal, subcutaneous, or intramuscular application for secondary immunization.

10. The use according to claim 7, 8, or 9,
**characterized in that**
for secondary immunization said pharmaceutical preparation contains the following amounts:
a) 1-200 x 10⁶ inactivated tumor cells, preferably 1-100 x 10⁶ inactivated tumor cells;
b) 10-500 x 10⁶ immune cells, preferably 50-200 x 10⁶ immune cells;
c) 1-10 µg trifunctional bispecific antibodies or trispecific antibodies, preferably 2-5 µg antibodies.

11. The use according to one or more of the preceding claims,
**characterized in that**
an intact trifunctional bispecific or trispecific antibody is used.

12. The use according to one or more of the preceding claims,
**characterized in that**
said antibody is capable of binding to Fc receptor-positive cells having an Fcγ receptor I or III.

13. The use according to one or more of the preceding claims,
**characterized in that**
said antibodies are capable of binding to monocytes, macrophages, dendritic cells, "natural killer" cells (NK cells) and/or activated neutrophil cells as the Fcγ receptor I- and/or III-positive cells.

14. The use according to one or more of the preceding claims,
**characterized in that**
said antibodies are capable of inducing tumor-reactive complement-binding antibodies and therefore of inducing a humoral immune response.

15. The use according to one or more of the preceding claims,
**characterized in that**
said antibodies are selected to bind to the T cell via CD2, CD3, CD4, CD5, CD6, CD8, CD28, CD40L (= CD154) and/or CD44.

16. The use according to one or more of the preceding claims,
**characterized in that**
said antibodies are selected to initiate or increase, after their binding to the Fc receptor-positive cells, the expression of CD40, CD80, CD86, ICAM-1, and/or LFA-3 as co-stimulatory antigens, or/and the secretion of cytokines by the Fc receptor-positive cell.

17. The use according to one or more of the preceding claims,
**characterized in that**
said antibodies are selected to increase the secretion of the cytokines IL-1, IL-2, IL-4, IL-6, IL-8, IL-12, INF-γ and/or TNF-α.

18. The use according to one or more of the preceding claims,
**characterized in that**
said bispecific antibody is selected to be an anti-CD3 X anti-tumor-associated antigen antibody and/or anti-CD4 X anti-tumor-associated antigen antibody and/or anti-CD5 X anti-tumor-associated antigen antibody and/or anti-CD6 X anti-tumor-associated antigen antibody and/or anti-CD8 X anti-tumor-associated antigen antibody and/or anti-CD2 X anti-tumor-associated antigen antibody and/or anti-CD28 X anti-tumor-associated antigen antibody and/or anti-CD40L X anti-tumor-associated antigen antibody and/or anti-CD44 X anti-tumor-associated antigen antibody.

19. The use according to one or more of the preceding claims,
**characterized in that**
said bispecific antibody is selected from a heterologous bispecific or trispecific antibody, preferably from a heterologous rat/mouse bispecific antibody.

20. The use according to one or more of the preceding claims,
**characterized in that**
said trispecific antibody has one T cell binding arm, one tumor cell binding arm, and a third specificity for binding to Fc receptor-positive cells

21. The use according to one or more of the preceding claims,
**characterized in that**
said trispecific antibody is selected to be an anti-CD3 X anti-tumor-associated antigen antibody and/or anti-CD4 X anti-tumor-associated antigen antibody and/or anti-CD5 X anti-tumor-associated antigen antibody and/or anti-CD6 X anti-tumor-associated antigen antibody and/or anti-CD8 X anti-tumor-associated antigen antibody and/or anti-CD2 X anti-tumor-associated antigen antibody and/or anti-CD28 X anti-tumor-associated antigen antibody and/or anti-CD40L X anti-tumor-associated antigen antibody and/or anti-CD44 X anti-tumor-associated antigen antibody.

22. The use according to one or more of the preceding claims 7, 8, and 10,
**characterized in that**
tumor cells are used which have been treated by irradiation, preferably by γ-irradiation, further preferred in a dose intensity of 50 to 200 Gy, or by chemical substances, preferably by mitomycin C.

23. The use according to one or more of the preceding claims 7, 8, and 10,
**characterized in that**
for enhancing the immunogenicity of the tumor cells they are subjected to a heat pretreatment prior to administration.

24. The use according to one or more of the preceding claims,
**characterized in that**
said antibody is further selected to be capable of activating Fc receptor-positive cells whereby the expression of cytokines and/or co-stimulatory antigens is initiated or increased.

25. The use according to one or more of the preceding claims,
**characterized in that**
autologous immune cells from PBMC, apherisates or bone marrow are employed.

26. The use according to one or more of the preceding claims,
**characterized in that**
said bispecific and/or trispecific antibody is present in an amount of 0.1 - 500 µg, the autologous or allogenic tumor cells are present in an amount of 10⁵ - 2 x 10⁸, and the autologous immune cells are present in an amount of 5 x 10⁶ - 5 x 10⁸ cells.

27. The use according to one or more of the preceding claims,
**characterized in that**
in addition to the destruction of the tumor cells there is established an immunity against the tumor cells to which the trifunctional bispecific antibodies or trispecific antibodies are directed.

## Revendications

1. Utilisation d'un anticorps trifonctionnel bispécifique et/ou trispécifique possédant les propriétés suivantes :
a) liaison à une cellule T ;
b) liaison à au moins un antigène sur une cellule tumorale, qui est associée à une ascite maligne et/ou à un épanchement pleural ;
c) liaison à des cellules positives pour le récepteur Fc, par son fragment Fc (dans le cas d'anticorps bispécifiques), ou par une troisième spécificité (dans le cas d'anticorps trispécifiques),
pour la fabrication d'une composition pharmaceutique pour le traitement d'ascites malignes et/ou d'un épanchement pleural, en vue de la destruction des cellules tumorales dans le liquide ascitique et/ou dans l'épanchement pleural.

2. Utilisation selon la revendication 1, **caractérisée en ce que** la préparation pharmaceutique est destinée à être administrée sous la forme d'une première dose et d'au moins une dose supplémentaire.

3. Utilisation selon la revendication 1 ou 2, **caractérisée en ce que** la préparation pharmaceutique à administrer sous la forme d'une deuxième dose est destinée à l'activation et à la prolifération des cellules immunitaires et sous la forme d'au moins une troisième dose est destinée à la destruction des cellules tumorales.

4. Utilisation selon une ou plusieurs des revendications précédentes, **caractérisée en ce que** la préparation pharmaceutique à administrer sous la forme d'une première dose est destinée à contrôler les réactions allergiques contre les anticorps administrés.

5. Utilisation selon une ou plusieurs des revendications précédentes, **caractérisée en ce que** la préparation pharmaceutique est conçue de telle sorte que la première dose à administrer le premier jour contient 1 à 20 µg, de préférence 5 à 10 µg de l'anticorps, la deuxième dose à administrer contient une quantité de 20 à 100 µg, de préférence 30 à 60 µg de l'anticorps, et chaque dose supplémentaire à administrer contient une quantité de 100 à 500 µg, de préférence 100 à 300 µg de l'anticorps.

6. Utilisation selon une ou plusieurs des revendications précédentes, **caractérisée en ce que** l'anticorps est administré en 3 à 8 doses, de préférence 3 à 6 doses pour une immunisation primaire, de préférence un intervalle de temps de 2 à 3 jours étant prévu entre chaque administration et, de plus, l'anticorps peut être administré de préférence jusqu'à ce qu'une réaction contre l'anticorps administré soit décelable.

7. Utilisation selon une ou plusieurs des revendications précédentes, **caractérisée en ce que** la préparation pharmaceutique est conçue de telle sorte que pour une immunisation secondaire pendant l'administration des autres doses, celles-ci contiennent des cellules tumorales inactivées autologues et/ou allogéniques et des cellules immunitaires autologues.

8. Utilisation selon la revendication 7, **caractérisée en ce que** les cellules immunitaires autologues utilisées sont des cellules d'aphérèse, qui sont prélevées de préférence avant la première chimiothérapie.

9. Utilisation selon une ou plusieurs des revendications précédentes, **caractérisée en ce que** la préparation pharmaceutique pour l'immunisation primaire est conçue de préférence pour une administration intrapéritonéale et celle pour l'immunisation secondaire est conçue pour une administration intradermique, sous-cutanée ou intramusculaire.

10. Utilisation selon la revendication 7, 8 ou 9, **caractérisée en ce que** la préparation pharmaceutique pour l'immunisation secondaire contient les quantités suivantes :
a) 1 à 200 x 10⁶ cellules tumorales inactivées, de préférence 10 à 100 x 10⁶ cellules tumorales inactivées,
b) 10 à 500 x 10⁶ cellules immunitaires, de préférence 50 à 200 x 10⁶ cellules immunitaires,
c) 1 à 10 µg d'anticorps trifonctionnels bispécifiques ou d'anticorps trispécifiques, de préférence 2 à 5 µg d'anticorps.

11. Utilisation selon une ou plusieurs des revendications précédentes, **caractérisée en ce que** l'on utilise un anticorps intact trifonctionnel bispécifique ou trispécifique.

12. Utilisation selon une ou plusieurs des revendications précédentes,
**caractérisée en ce que** l'anticorps est apte à être lié à un récepteur Fc de cellules positives, qui contiennent un récepteur Fcγ I ou III.

13. Utilisation selon une ou plusieurs des revendications précédentes, **caractérisée en ce que** les anticorps sont aptes à être liés à des monocytes, des macrophages, des cellules dendritiques, des cellules NK dites « cellules tueuses naturelles » et/ou des cellules neutrophiles activées sous forme de récepteur Fcγ I et/ou III de cellules positives.

14. Utilisation selon une ou plusieurs des revendications précédentes, **caractérisée en ce que** les anticorps sont aptes à induire des anticorps à liaison complémentaire réactifs à la tumeur et, de ce fait, à induire une réponse immunitaire humorale.

15. Utilisation selon une ou plusieurs des revendications précédentes, **caractérisée en ce que** les anticorps sont choisis de telle sorte qu'ils se lient aux cellules T par l'intermédiaire de CD2, CD3, CD4, CD5, CD6, CD8, CD28, CD40L (= CD154) et/ou CD44.

16. Utilisation selon une ou plusieurs des revendications précédentes, **caractérisée en ce que** les anticorps sont choisis de telle sorte que, après leur liaison aux cellules positives pour le récepteur Fc, l'expression de CD40, CD80, CD86, ICAM-1 et/ou LFA-3 en tant qu'antigènes co-stimulateurs et/ou la sécrétion des cytokines est initiée ou accrue par les cellules positives pour le récepteur Fc.

17. Utilisation selon une ou plusieurs des revendications précédentes, **caractérisée en ce que** les anticorps sont choisis de telle sorte que la sécrétion de cytokine IL-1, IL-2, IL-4, IL-6, IL-8, IL-12, INF-γ et/ou de TNF-α est accrue.

18. Utilisation selon une ou plusieurs des revendications précédentes, **caractérisée en ce que** l'anticorps bispécifique est un anticorps, qui est choisi de telle sorte qu'il est un anticorps anti-CD3 X anti-antigène associé à une tumeur et/ou un anticorps anti-CD4 X anti-antigène associé à une tumeur et/ou un anticorps anti-CD5 X anti-antigène associé à une tumeur et/ou un anticorps anti-CD6 X anti-antigène associé à une tumeur et/ou un anticorps anti-CD8 X anti-antigène associé à une tumeur et/ou un anticorps anti-CD2 X anti-antigène associé à une tumeur et/ou un anticorps anti-CD28 X anti-antigène associé à une tumeur et/ou un anticorps anti-CD40L X anti-antigène associé à une tumeur et/ou un anticorps anti-CD44 X anti-antigène associé à une tumeur.

19. Utilisation selon une ou plusieurs des revendications précédentes, **caractérisée en ce que** l'anticorps bispécifique est choisi parmi un anticorps hétérologue bispécifique ou trispécifique, de préférence un anticorps bispécifique hétérologue rat/souris.

20. Utilisation selon une ou plusieurs des revendications précédentes, **caractérisée en ce que** l'anticorps trispécifique comporte un bras de liaison aux cellules T, un bras de liaison aux cellules tumorales et une troisième spécificité pour la liaison des cellules positives pour le récepteur Fc.

21. Utilisation selon une ou plusieurs des revendications précédentes, **caractérisée en ce que** l'anticorps trispécifique est choisi de telle sorte qu'il est un anticorps anti-CD3 X anti-antigène associé à une tumeur et/ou un anticorps anti-CD4 X anti-antigène associé à une tumeur et/ou un anticorps anti-CD5 X anti-antigène associé à une tumeur et/ou un anticorps anti-CD6 X anti-antigène associé à une tumeur et/ou un anticorps anti-CD8 X anti-antigène associé à une tumeur et/ou un anticorps antigène anti-CD28 X anti-antigène associé à une tumeur et/ou un anticorps anti-CD40L X anti-antigène associé à une tumeur et/ou un anticorps anti-CD44 X anti-antigène associé à une tumeur.

22. Utilisation selon une ou plusieurs des revendications précédentes 7, 8 et 10, **caractérisée en ce que** l'on utilise des cellules tumorales qui ont été traitées par radiation, de préférence par radiation γ, de manière encore plus préférée avec une dose de radiation de l'ordre de 50 à 200 Gy, ou par des substances chimiques, de préférence la mitomycine C.

23. Utilisation selon une ou plusieurs des revendications précédentes 7, 8 et 10, **caractérisée en ce que** pour accroître l'immunogénicité des cellules tumorales, celles-ci sont soumises à un traitement thermique préliminaire avant leur administration.

24. Utilisation selon une ou plusieurs des revendications précédentes, **caractérisée en ce que**, en outre, l'anticorps est choisi de telle sorte qu'il est apte à activer les cellules positives pour le récepteur Fc, moyennant quoi l'expression des cytokines et/ou des antigènes de co-stimulation est initiée ou accrue.

25. Utilisation selon une ou plusieurs des revendications précédentes, **caractérisée en ce que** l'on utilise des cellules immunitaires autologues de PBMC, de produits d'aphérèse ou de moelle osseuse.

26. Utilisation selon une ou plusieurs des revendications précédentes, **caractérisée en ce que** l'anticorps bispécifique et/ou trispécifique est contenu en une quantité de 0,1 à 500 µg, les cellules tumorales autologues ou allogènes en une quantité de 10⁵ à 2 x 10⁸ et les cellules immunitaires autologues en une quantité de 5 x 10⁶ à 5 x 10⁸ de cellules.

27. Utilisation selon une ou plusieurs des revendications précédentes, **caractérisée en ce que**, en plus de la destruction des cellules tumorales, il est établi une immunité contre les cellules tumorales, contre lesquelles sont dirigés les anticorps trifonctionnels bispécifiques ou trispécifiques.
